# EUROPEAN PATENT SPECIFICATION

(11) **EP 1 748 779 B1**
(45) Date of publication and mention of the grant of the patent: **08.09.2010**
(21) Application number: 05738733.4
(22) Date of filing: 28.04.2005
(51) Int. Cl.: A61K 31/5685, A61K 9/70, A61P 19/10

(54) **TRANSDERMAL STEROID FORMULATION**
TRANSDERMALE STEROIDFORMULIERUNG
PREPARATION STEROIDIENNE TRANSDERMIQUE

(30) Priority: 28.04.2004 GB 0409498
(43) Date of publication of application: 07.02.2007
(73) Proprietor: Hunter-Fleming Limited, Bristol BS1 6EA (GB)
(72) Inventor: MURRAY, James, Robert Regus House, Bristol BS1 6EA (GB); KAMIYAMA, Fumio CosMed Ltd, Rale YamashinaSkyheiz, Yamashina-ku, Kyoto 607-8161 (JP); WÜLFERT, Ernst, B-1180 Brussels (BE)
(74) Representative: Mancini, Vincenzo
(86) International application number: PCT/GB2005/001596
(87) International publication number: WO 2005/105104

(56) References cited:
- WO-A-96/36339
- US-A1- 2001 023 261
- HENZL M R ET AL: "TRANSDERMAL DELIVERY OF SEX STEROIDS FOR HORMONE REPLACEMENT THERAPY AND CONTRACEPTION A REVIEW OF PRINCIPLES AND PRACTICE" JOURNAL OF REPRODUCTIVE MEDICINE, JOURNAL OF REPRODUCTIVE MEDICINE, INC., CHICAGO,, US, vol. 48, no. 7, July 2003 (2003-07), pages 525-540, XP009020455 ISSN: 0024-7758
- SITRUK-WARE R: "TRANSDERMAL DELIVERY OF STEROIDS" CONTRACEPTION, vol. 39, no. 1, 1989, pages 1-20, XP002343831 ISSN: 0010-7824

## Description

The present invention relates to a transdermal formulation for the treatment, inter alia, of osteoporosis and related ailments.

Osteoporosis, a condition in which bone mass decreases, resulting in the bone losing some of its structural integrity, is a condition particularly prevalent in postmenopausal women. It is often treated by the administration of steroids, such as oestrogen.

EP698612 discloses that certain 11-hydroxy steroids are particularly useful for the treatment and prevention of osteoporosis and for osteogenesis, without many of the side effects of known treatments. There is no indication in this specification as to the route by which the steroids are to be administered. However, the Examples show the use of the compounds by injection.

WO 02/00224 and WO 02/00225 disclose the use of certain 7-hydroxy-steroid compounds, especially 3-hydroxy-7β-hydroxy-steroid compounds, for protection against neuronal cell death. These suggest that, in order to achieve a rapid effect, the compounds should be administered by intravenous injection.

WO 03/015791 discloses the use of 3-hydroxy-7-hydroxy steroids and 3-oxo-7-hydroxy steroids, especially the 7β-isomers thereof, and pharmaceutically acceptable esters thereof for protection against ischaemia-induced damage to peripheral organs, such as the heart or kidneys.

Although the two most common methods of administering medication are orally or by injection, both methods have serious disadvantages. Oral administration of a drug depends on the drug being stable to the acid environment of the stomach as well as to the intestinal mechanism, or on the drug being protected from that environment. Moreover, people differ in the rate and manner in which drugs are absorbed, and so the amount of the drug actually reaching the bloodstream via the oral route can vary enormously from person to person.

Injection avoids these disadvantages but has to be done by a skilled or brained person, is often disliked, sometimes intensely, by the patient and can cause damage if done too often.

Traditionally, transdermal administration (i.e. administration through the skin, but without breaking the skin) has been used only for those drugs which affect the skin or immediately underlying muscle tissue. However, it can be used with other drugs provided that those drugs can pass through the skin sufficiently efficiently and sufficiently predictably. Documents WO 96/36 339 and US 2001/0023 261 disclose the transdermal administration of steroids, encompassed by the application. These documents do not disclose the adhesive composition as claimed.

We have now surprisingly found an adhesive patch or adhesive composition for transdermal delivery of certain steroids, including those of EP698612, WO 02/00224 and WO 02/00225.

Thus, the present invention consists in an adhesive patch for the transdermal administration of a compound of formula (I): 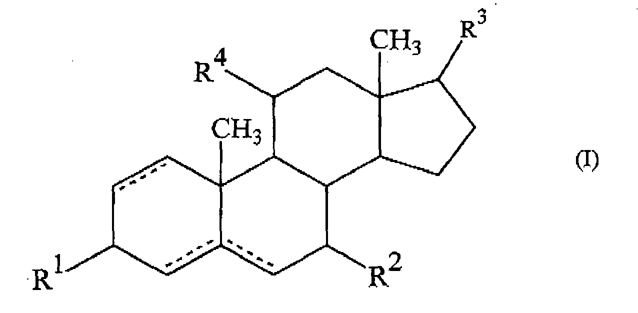 in which R¹; R², R³ and R⁴ are the same as or different from each other and each represents an oxo group, a hydroxy group, a mercapto group, a hydrogen atom, a halogen atom, an alkoxy group or an aryloxy group; and the dotted line indicates that there may be a single or double bond between one of the respective pairs of carbon atoms or an ester thereof, said patch comprising a substrate and a layer of adhesive on the substrate, in which said adhesive comprises a copolymer of from 40 to 60% by weight of methoxyethyl acrylate, from 30 to 40% by weight of lauryl acrylate or methacrylate and from 10 to 25% by weight of a polar monomer, said adhesive having dispersed therein at least one compound of formula (I) or an ester thereof.

The invention further consists in an adhesive composition, comprising an adhesive having at least one compound of formula (I) or an ester thereof dispersed therein, wherein said adhesive having dispersed therein at least one compound of formula (I) or an ester thereof is as defined above, for the transdermal administration of said compound or ester.

Among the compounds of the present invention, preferred compounds include those compounds of formula (II): 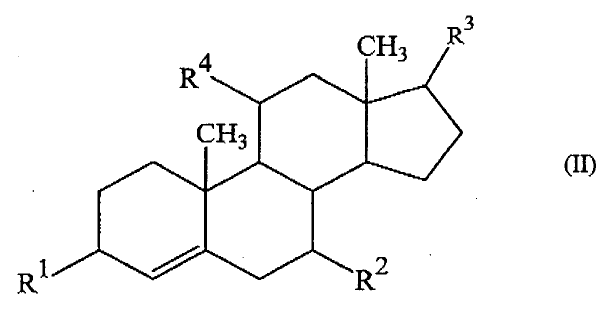 in which R¹, R², R³ and R⁴ are as defined above and esters thereof.

Another preferred class of compounds of the present invention are those compounds of formula (III): 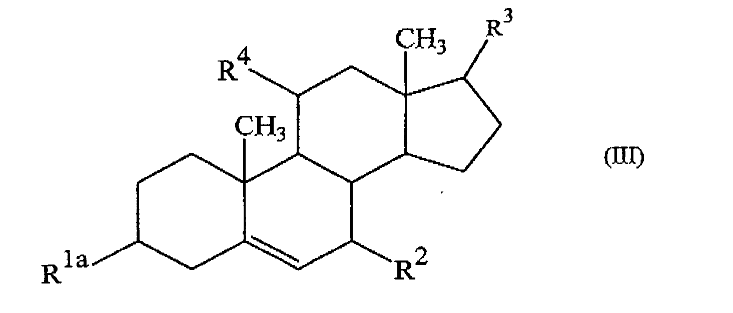 in which R^{1a} represents an oxo group, a hydroxy group, a mercapto group or a halogen atom; and R², R³ and R⁴ are as defined above and esters thereof.

A still further preferred class of compounds of the present invention are those compounds of formula (IV): 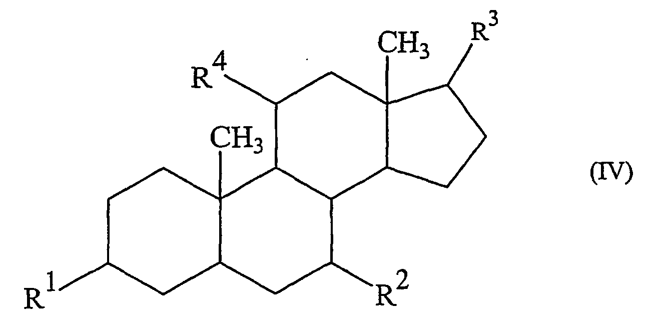 in which R¹, R², R³ and R⁴ are as defined above and esters thereof.

Examples of compounds of formula (II) include 11β-hydroxy-4-androstene-3,17-dione, which has the formula (IIa): 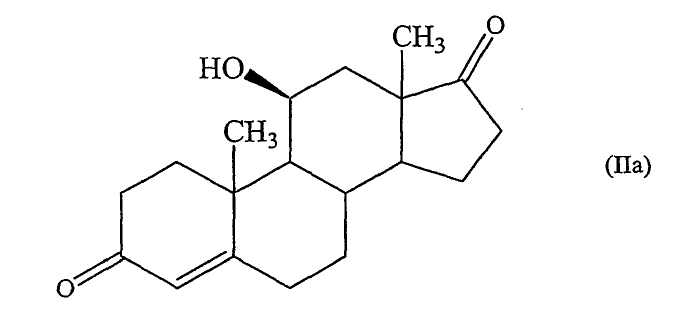 and esters thereof. This compound, which is the preferred compound for use in the present invention, is hereinafter referred to as "HAD".

Examples of compounds of formula (III) include 7α-hydroxy-dehydroepiandrosterone (7α-hydroxy-DHEA), which has the formula (IIIa): 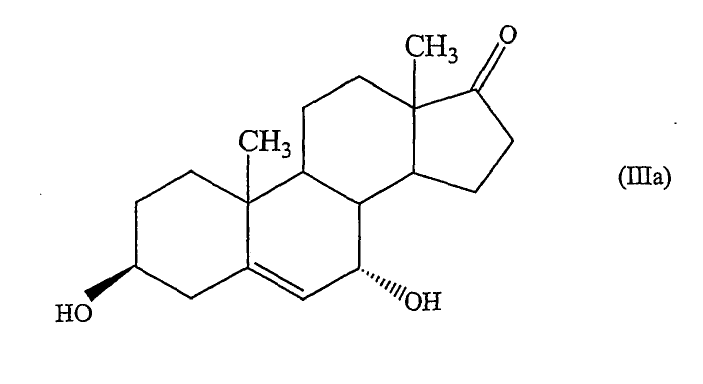 and esters thereof.

Examples of compounds of formula (IV) include 7β-hydroxy-epiandrosterone, hereinafter referred to as 7β-OH EPIA, which has the formula (IVa): 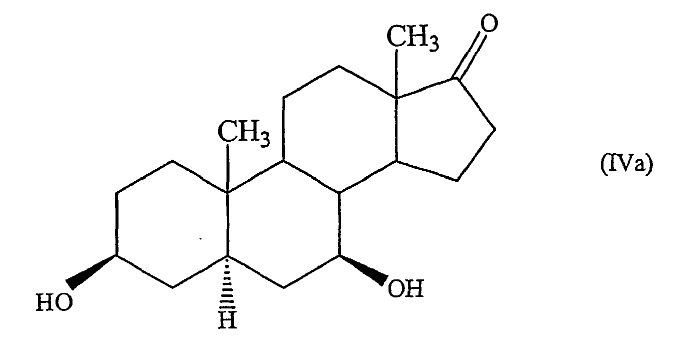 and esters thereof.

In the compounds of the present invention, where R¹, R^{1a}, R², R³ or R⁴ represents a halogen atom, this may be a fluorine, chlorine, bromine or iodine atom, preferably a chlorine atom.

Other useful steroids which may be used as the compound of formula (I) include such androgens as testosterone and 3β,17β-dihydroxyandrostane.

Of the compounds referred to above, the most preferred are 7α-hydroxy-dehydroepiandrosterone, 7β-hydroxy-epiandrosterone (7β-OH EPIA) and 11β-hydroxy-4-androstene-3,17-dione (HAD).

Patches for transdermal administration of medication normally comprise a substrate of a medically acceptable fabric or film on which is a layer of an adhesive impregnated with the medication. Any substrate commonly used in the field may be used in the present invention. It may, for example, be a woven fabric, non-woven fabric, porous film or moulded film, ideally 10-100µm thick. Woven fabrics, non-woven fabrics and porous films are useful in that they allow the passage of water vapour, while moulded films are useful for providing a barrier to bacteria and for waterproofing.

However, it will be appreciated that the nature of the adhesive may have a significant effect on the utility of the patch, and so this needs to be chosen with some care. Thus, the adhesive must be capable of adhering to the skin, it must permit sustained drug release and it must not cause irritation to the skin. In general, it must maintain these properties over a relatively long period, e.g. 3 to 7 days, after attachment, despite the normal flexing of the skin and possibly washing. Moreover, the adhesive must not be so strong that it causes damage to the skin when the patch is removed.

We have now found that a particular class of adhesives is especially useful with the steroid compounds of formula (I) and esters thereof used in the present invention.

These new adhesives comprise a copolymer of from 40 to 60% by weight of methoxyethyl acrylate, from 30 to 40% by weight of lauryl acrylate or lauryl methacrylate and from 10 to 25% by weight of a polar monomer.

The amount of units derived from methoxyethyl acrylate in the copolymer should be from 40 to 60% by weight, more preferably from 45 to 55% by weight. If this amount is greater than 60% by weight, gelling occurs during polymerisation and the resulting copolymer becomes insoluble. Moreover, the viscosity of the copolymer is poor. On the other hand, if the amount of methoxyethyl methacrylate is less than 40%, drug solubility in the adhesive is reduced and the relatively high drug loadings otherwise achievable are not achieved.

The second co-monomer, lauryl acrylate and/or lauryl methacrylate, is present in an amount of from 30 to 40% by weight of the monomer units in the copolymer. Its presence enhances the adhesion of the adhesive and, when combined with the methoxyethyl acrylate, it provides suitable conditions for dissolving the steroids used in the present invention as well as absorption promoters, where these are used. If the amount of lauryl acrylate and/or lauryl methacrylate units in the copolymer is less than 30% by weight, adequate hydrophobicity is not achieved. On the other hand, if the amount is greater than 40%, the copolymer becomes too hydrophobic and drug solubility is reduced.

If 2-ethylhexyl acrylate or 2-ethylhexyl methacrylate were used in place of the lauryl analogue, since the chain length of the 2-ethylhexyl group is less than that of the lauryl group, the ability of the resulting copolymer to dissolve hydrophobic drugs and absorption promoters would be reduced. On the other hand, if stearyl acrylate or methacrylate, with a longer chain length, were used in place of the lauryl analogue, the adhesion of the adhesive would be poor.

The third necessary monomer in the copolymers used in the present invention is a polar monomer. This serves to impart to the copolymer higher cohesion without damaging the other desirable properties mentioned above and it can improve the cross-linking of the polymer. It should be present in the copolymer in an amount of from 10 to 25% by weight of the copolymer units. Less than 10% by weight of the polar monomer will not give adequate cohesion, while more than 25% will result in the copolymer becoming too polar and the adhesiveness being reduced. Examples of suitable polar monomers include acrylic acid, methacrylic acid, acrylamide, methacrylamide, N-vinyl-2-pyrrolidone, N,N-dimethylacrylamide, 2-hydroxyethyl acrylate and vinyl acetate. A single one of these or a combination of two or more may be used. Of these, acrylic acid, N-vinyl-2-pyrrolidone and 2-hydroxyethyl acrylate are preferred, N-vinyl-2-pyrrolidone being most preferred, either alone or in combination with at least one other polar monomer. In any event, we particularly prefer that units derived from N-vinyl-2-pyrrolidone should constitute at 5% by weight of the copolymer, provided that the total amount of polar monomer is, as stated above, from 10 to 25% by weight of the copolymer.

As mentioned above, an absorption promoter may be included in the adhesive composition according to the present invention. Examples of such absorption promoters include: fatty acid esters, such as isopropyl palmitate and isopropyl myristate; glycerol esters, such as glyceryl monolaurate and glyceryl monooleate; acid amides, such as lauric acid diethanolamide; and neutral surfactants, such as polyethylene glycol dilauryl ether. However, such materials are well known in the art, and any common absorption promoter may be used. The preferred absorption promoter is isopropyl myristate. Clearly, the amount of the absorption promoter must be sufficient to enhance transdermal absorption of the drug. However, too much may have the effect of reducing the adhesiveness of the patch. Accordingly, we prefer that the amount of absorption promoter should be from 3 to 40 parts by weight per 100 parts by weight of the copolymer.

It is also preferred that the copolymer used in the adhesive composition should be cross-linked by means of a cross-linking agent in order to enhance cohesion. Examples of such cross-linking agents include isocyanates and chelating agents. The amount of the cross-linking agent used is preferably from 0.1 to 2 parts by weight. If the amount used is less than 0.1 parts by weight, little cross-linking takes place and there is no significant benefit from its addition. On the other hand, if the amount added is more than 2 parts by weight, adhesiveness is reduced.

The copolymer may be prepared by methods well known in the art for the preparation of polymers of this type, for example by free radical polymerisation using the monomers defined above. Any conventional process, such as solution polymerisation, suspension polymerisation or emulsion polymerisation, may be used. Solution polymerisation is particularly preferred, since the resulting molecular weight distribution is relatively narrow and there is, as a result, little variability in adhesiveness.

Surprisingly, we have found that the copolymers suggested above, when used as the adhesive, actually enhance the absorption of the compounds of formula (I) through the skin.

The active ingredient may be mixed with the adhesive copolymer by adding it, and, if desired, an absorption promoter and/or a cross-linking agent, to a solution containing the copolymer in a suitable solvent. The resulting mixture may then be laminated onto the desired support using, for example, a knife or roll coater, and then dried in an oven, for example at a temperature of from 50 to 100°C, for a suitable period to remove the solvent and cause the adhesive composition containing the active ingredient to adhere to the substrate.

The amount of the mixture applied to the substrate is preferably such that the thickness of the composition after drying is from 30 to 120µm. If the thickness is less than 30µm, adhesion of the composition is weak and it may be difficult to incorporate an adequate amount of the active ingredient. On the other hand, if the amount is greater than 120µm, it is difficult to form a layer of the composition and to dry it.

The present invention is further illustrated by the following non-limiting Examples, of which Examples 1 to 3 illustrate the preparation of the adhesive copolymers and Examples 4, 5, 6 and Comparative Examples 1 to 10 illustrate the preparation and use of patches of the present invention.

### EXAMPLES 1-3

200 g of ethyl acetate (solvent), 0.05 g of azobisisobutyronitrile (initiator) and the monomers shown in the following Table 1 were charged into a reaction vessel, which was then sparged with nitrogen. Polymerisation was then carried out for 15 hours at 20°C. The resulting copolymer solution was coated with a knife coater onto a polyethylene terephthalate film to a dried thickness of 100µm, and then dried at a temperature of 90°C for 15 minute to produce adhesive sheets.

The resulting sheets were attached to the keratin layer of the shaven skin on the abdomen of Wistar rats. They were left there for 24 hours, after which they were removed, and the adhesiveness and state of the adhesive layer were observed with the naked eye. The results are shown in Table 1.

**Table 1**

| | **Monomer** | **Amount of monomer** | **Adhesion** | **Cohesion** |
|---|---|---|---|---|
| **Example 1** | methoxyethyl acrylate | 43 g | no detachment within 24 hours | no residue left on body |
| | lauryl acrylate | 38 g | | |
| | N-vinylpyrrolidone | 6 g | | |
| | acrylic acid | 3 g | | |
| | hydroxyethyl acetate | 10 g | | |
| **Example 2** | methoxyethyl acrylate | 48 g | no detachment within 24 hours | no residue left on body |
| | lauryl acrylate | 34 g | | |
| | N-vinylpyrrolidone | 15 g | | |
| | acrylic acid | 3 g | | |
| **Example 3** | methoxyethyl acrylate | 50 g | no detachment within 24 hours | no residue left on body |
| | lauryl acrylate | 36 g | | |
| | N-vinylpyrrolidone | 5 g | | |
| | hydroxyethyl acetate | 10 g | | |

### EXAMPLES 4 & 5 & COMPARATIVE EXAMPLES 1-10

### Preparation of adhesive patch

Into the adhesive solution (copolymer solution) of Example 1 and into the adhesive solutions used in the Comparative Examples, the drug specified below, and, where used, isopropyl myristate and/or a crosslinker, were added and dissolved. The resulting compositions containing the dissolved drug were coated onto a poly(ethylene terephthalate) film to give a thickness when dry of 100 µm, and dried at a temperature of 90°C for 15 minutes, to produce drug-containing adhesive sheets.

The adhesives used for the Comparative Examples were obtained from National Starch, and are sold under the designations DT2287, DT2516, DT2051, DT2052, and DT2074.

The compositions of the adhesive mixtures are shown in Tables 2 and 3.

**Table 2**

| **Example No.** | | **4** | **Comp 1** | **Comp 2** | **Comp 3** | **Comp 4** | **Comp 5** |
|---|---|---|---|---|---|---|---|
| Ingredient | | | | | | | |
| Example No. 1 | Solid, mg | 250 | - | - | - | - | - |
| DT2287 | Solid, mg | - | 250 | - | - | - | - |
| DT2516 | Solid, mg | - | - | 250 | - | - | - |
| DT2051 | Solid, mg | - | - | - | 250 | - | - |
| DT2052 | Solid, mg | - | - | - | - | 250 | - |
| DT2074 | Solid, mg | - | - | - | - | - | 250 |
| IPM | mg | 100 | - | - | - | 100 | 100 |
| HAD | mg | 7.0 | 5.0 | 7.5 | 7.0 | 6.0 | 5.5 |
| Crosslinker* | mg | 250 | - | - | - | - | - |

| | | | | | | | |
|---|---|---|---|---|---|---|---|
| * 0.05% of aluminium acetylacetate/tetrahydrofuran solution. | | | | | | | |

**Table 3**

| **Example No.** | | **5** | **Comp 6** | **Comp 7** | **Comp 8** | **Comp 9** | **Comp 10** |
|---|---|---|---|---|---|---|---|
| Ingredient | | | | | | | |
| Example No. 1 | Solid, mg | 250 | - | - | - | - | - |
| DT2287 | Solid, mg | - | 250 | - | - | - | - |
| DT2516 | Solid, mg | - | - | 250 | - | - | - |
| DT2051 | Solid, mg | - | - | - | 250 | - | - |
| DT2052 | Solid, mg | - | - | - | - | 250 | - |
| DT2074 | Solid, mg | - | - | - | - | - | 250 |
| IPM | mg | 100 | - | 100 | - | 100 | 100 |
| 7β-OH EPIA | mg | 8.0 | 6.5 | 12.0 | 10.5 | 7.0 | 9.0 |
| Crosslinker* | mg | 0.002 | - | - | - | - | - |
| PEG 400 | mg | 30 | - | - | - | - | - |

| | | | | | | | |
|---|---|---|---|---|---|---|---|
| * 0.05% of aluminium acetylacetate/tetrahydrofuran solution. | | | | | | | |

As shown in Table 2 and Table 3, the various adhesives were loaded with the saturation concentration of HAD or 7β-OH EPIA. Where possible, isopropyl myristate (IPM) was used as an enhancer, since it is an excellent pharmaceutical excipient for the transdermal delivery of a drug because of its marked enhancing effect and low skin irritation. However, most of the conventional adhesives could not load enough IPM because it resulted in a decrease in cohesion.

### EXAMPLE 6

### 1. Saturation concentration of HAD in adhesives

The adhesive patches described in Example 4 and Comparative Examples 1-5 were mixed with progressively increasing concentrations of HAD and were stored (adhesive side up) at room temperature for 8 weeks. At the end of this time, the patches were examined to determine whether HAD had crystallised on the surfaces of the patches. The saturation concentrations of HAD in the adhesives are shown in Table 4.

**Table 4**

| **Adhesives** | **Saturation of HAD (% of patch weight)** |
|---|---|
| Example 4 | 2.0 |
| Comp 1 | 2.0 |
| Comp 2 | 3.0 |
| Comp 3 | 2.0 |
| Comp 4 | 1.5 |
| Comp 5 | 2.0 |

### 2. Saturation concentration of 7β-OH EPIA in adhesives

The adhesive patches described in Example 5 and Comparative Examples 6-10 were mixed with progressively increasing concentrations of 7β-OH EPIA and were stored (adhesive side up) at room temperature for 8 weeks. At the end of this time, the patches were examined to determine whether 7β-OH EPIA had crystallised on the surfaces of the patches. The saturation concentrations of 7β-OH EPIA in the adhesives are shown in Table 5.

**Table 5**

| | |
|---|---|
| **Adhesives** | **Saturation of 7β-OH EPIA (% of patch weight)** |
| Example 5 | 2.0 |
| Comp 6 | 2.5 |
| Comp 7 | 3.3 |
| Comp 8 | 4.0 |
| Comp 9 | 2.0 |
| Comp 10 | 2.5 |

### 3. Permeation behaviour

The materials used were as follows:

| | |
|---|---|
| *Animals:* | *Wistar rats (male, body weight 240 ∼ 250g)* |
| *Test patches:* | *Examples 4 and 5 and Comparative Examples 1-10* |
| | *Commercial estradiol patch as permeation marker (Estrana, Hisamitsu Pharmaceutical Co.)* |
| *Patch size:* | *8 mm diameter for HAD and 10mm diameter for 7*β*-OHEPIA* |
| *Receptor solution:* | *PEG400:water (1:3)* |
| *HPLC conditions* | |

| | | |
|---|---|---|
| *Column* | *ODS* | *5 um, 4. 6 x 150 mm* |
| *Mobile* | *water : acetonitrile* | *(55:45)* |
| *Flow rate* | *1 ml*/*min* | |
| *UV detector* | *240 nm for HAD,* | |
| | *300nm for 7*β*-OH EPIA* | |
| *Column temperature* | *40°C* | |

The excised abdominal rat skin was mounted on a Franz type cell (receiver volume: 3ml) with a water jacket connected to a water bath at 37°C. The patch was adhered to the stratum corneum side of the skin. The receiver compartment was filled with a mixture of PEG400 and water (1:3 by volume) and stirred with a star head magnetic bead driven by a constant speed motor. At predetermined times, 100µl of sample was withdrawn from the receiver compartment, and the same volume of receptor solution was added to keep the volume constant. The drug concentration was analysed by HPLC.

For all adhesives, the amount of the drug which had permeated increased steadily with time. The results after 24 hours are shown in Table 6 and Table 7.

**Table 6**

| **Adhesive** | **Permeated amount** µ**g/cm²** |
|---|---|
| Example 4 | 30 |
| Comp 1 | 28 |
| Comp 2 | 20 |
| Comp 3 | 30 |
| Comp 4 | 50 |
| Coma 5 | 60 |
| Estradiol | 15 |

**Table 7**

| **Adhesive** | **Permeated amount** µ**g/cm²** |
|---|---|
| Example 5 | 162 |
| Comp 6 | 150 |
| Comp 7 | 110 |
| Comp 8 | 52 |
| Comp 9 | 74 |
| Comp 10 | 73 |
| Estradiol | 13 |

All patches showed that a much higher amount of HAD and 7β-OH EPIA had permeated than that of the permeation marker, estradiol. This means that HAD 7β-OH EPIA have a potential skin permeability.

It was surprisingly found that the skin permeation of HAD was best with adhesives having the functional group -COOH or a combination of the functional groups -COOH/-OH, which make the adhesive highly hydrophilic. This allows HAD to be released into the skin. Adhesives having just the -OH group, such as DT2516, did not allow good permeation, whereas adhesives with a combination of groups -COOH / - OH, such as Example 1 and DT2074, allowed good permeation. Allowing for experimental variations due to variations in individual rats, the adhesive of Example 1 is as good as, if not better than, the known adhesives DT2052 and DT2074 for HAD permeation.

### 4. Permeation in human skin

The protocol in 3 above was repeated with HAD on human skin using the composition of Example 4 (Table 2) together with 5% w/w of lactic acid as enhancer. A penetration flux of 1.1µg/cm²/hr in human skin was achieved.

## Claims

1. An adhesive patch for the transdermal administration of a compound of formula (I): 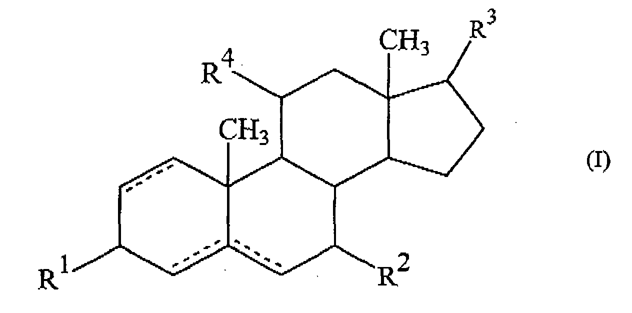 in which R¹; R², R³ and R⁴ are the same as or different from each other and each represents an oxo group, a hydroxy group, a mercapto group, a hydrogen atom, a halogen atom, an alkoxy group or an aryloxy group; and the dotted line indicates that there may be a single or double bond between one of the respective pairs of carbon atoms or an ester thereof, said patch comprising a substrate and a layer of adhesive on the substrate, in which said adhesive comprises a copolymer of from 40 to 60% by weight of methoxyethyl acrylate, from 30 to 40% by weight of lauryl acrylate or methacrylate and from 10 to 25% by weight of a polar monomer, said adhesive having dispersed therein at least one compound of formula (I) or an ester thereof.

2. A patch according to Claim 1, in which said compound is a compound of formula (II): 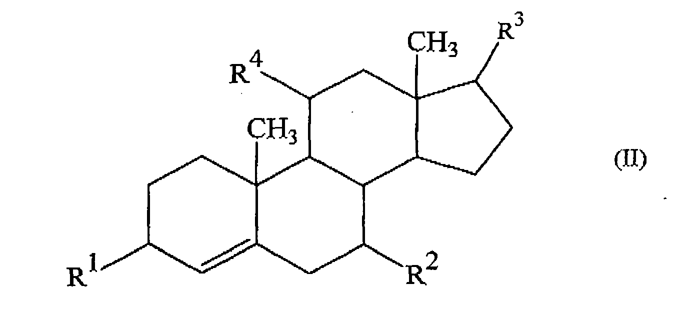 in which R¹, R², R³ and R⁴ are as defined in Claim 1 or an ester thereof.

3. A patch according to Claim 1, in which said compound is a compound of formula (III): 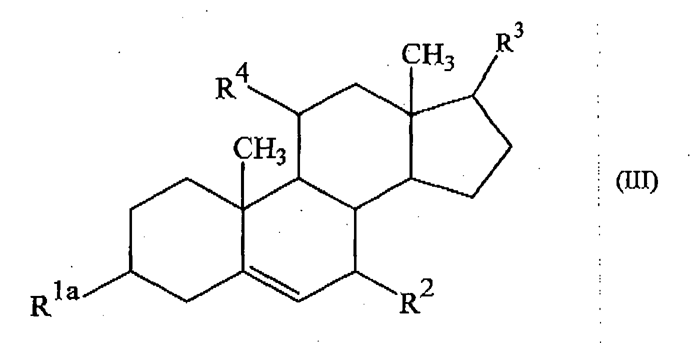 in which R^{1a} represents an oxo group, a hydroxy group, a mercapto group or a halogen atom; and R², R³ and R⁴ are as defined in Claim 1 or an ester thereof.

4. A patch according to Claim 1, in which said compound is a compound of formula (IV): 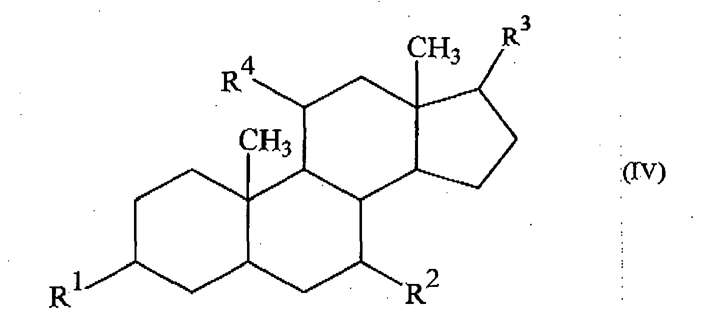 in which R¹, R², R³ and R⁴ are as defined in Claim 1 or an ester thereof.

5. A patch according to Claim 1, in which said compound is 11β-hydxoxy-4-androstene-3,17-dione.

6. A patch according to Claim 1, in which said compound is 7α-hydroxyy dehydroepiandrosterone.

7. A patch according to Claim 1, in which said compound is 7β-hydroxy-epiandrosterone.

8. A patch according to any one of Claims 1 to 7, in which the amount of units derived from methoxyethyl acrylate in the copolymer is from 45 to 55% by weight.

9. A patch according to any one of Claims 1 to 8, in which the polar monomer is acrylic acid, methacrylic acid, acrylamide, methacrylamide, N-vinyl-2-pyrrolidone, N,N-dimethylacrylamide, 2-hydroxyethyl acrylate or vinyl acetate.

10. A patch according to Claim 9, in which the polar monomer is acrylic acid, N-vinyl-2-pyrrolidone or 2-hydroxyethyl acrylate.

11. A patch according to Claim 10, in which the polar monomer is N-vinyl-2-pyrrolidone.

12. A patch according to any one of Claims 1 to 11, in which the adhesive additionally contains an absorption promoter.

13. A patch according to Claim 12, in which said absorption promoter is isopropyl myristate.

14. A patch according to Claim 12 or Claim 13, in which said absorption promoter is present in an amount from 3 to 40 parts by weight per 100 parts by weight of the copolymer.

15. A patch according to any one of Claims 1 to 14, in which the adhesive layer has a thickness of from 30 to 120µm.

16. A patch according to any one of Claims 1 to 15, for use in the treatment or prevention by transdermal application of osteoporosis, osteogenesis, neuronal cell death, or ischaemia-induced damage to peripheral organs.

17. An adhesive composition, comprising an adhesive having at least one compound of formula (I) or an ester thereof dispersed therein, wherein said adhesive having dispersed therein at least one compound of formula (I) or an ester thereof is as defined in any one of Claims 1 to 14, for the transdermal administration of said compound or ester.

18. An adhesive composition according to Claim 17, for use in the treatment or prevention by transdermal application of osteoporosis, osteogenesis, neuronal cell death, or ischaemia-induced damage to peripheral organs.

## Patentansprüche

1. Klebepflaster für die transdermale Verabreichung einer Verbindung der Formel (I): 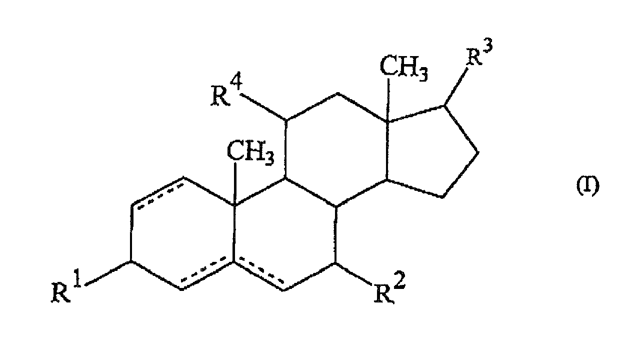 in der R¹, R², R³ und R⁴ gleich oder voneinander verschieden sind und jeweils eine Oxogruppe, eine Hydroxygruppe, eine Mercaptogruppe, ein Wasserstoffatom, ein Halogenatom, eine Alkoxygruppe oder eine Aryloxygruppe darstellen und die gestrichelte Linie anzeigt, dass eine Einfach- oder Doppelbindung zwischen einem der entsprechenden Paare von Kohlenstoffatomen sein kann, oder eines Esters davon, wobei das Pflaster ein Substrat und eine Klebstoffschicht auf dem Substrat umfasst, wobei der Klebstoff ein Copolymer aus 40 bis 60 Gew.-% Methoxyethylacrylat, 30 bis 40 Gew.-% Laurylacrylat oder -methacrylat und 10 bis 25 Gew.-% eines polaren Monomers umfasst, wobei der Klebstoff darin wenigstens eine Verbindung der Formel (I) oder einen Ester davon dispergiert hat.

2. Pflaster nach Anspruch 1, wobei die Verbindung eine Verbindung der Formel (II): 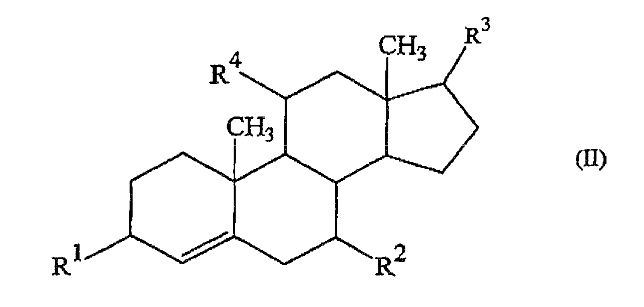 in der R¹, R², R³ und R⁴ wie in Anspruch 1 definiert sind, oder ein Ester davon ist.

3. Pflaster nach Anspruch 1, wobei die Verbindung eine Verbindung der Formel (III): 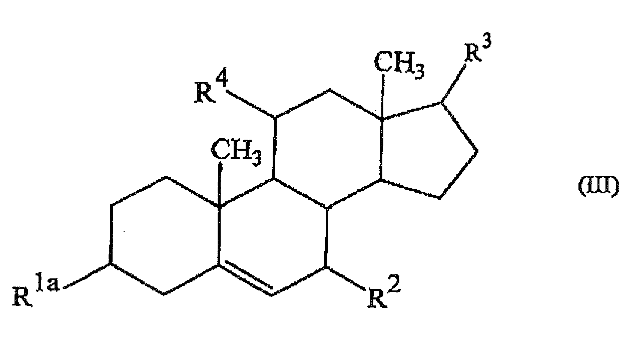 in der R^{1a} eine Oxogruppe, eine Hydroxygruppe, eine Mercaptogruppe oder ein Halogenatom darstellt und R², R³ und R⁴ wie in Anspruch 1 definiert sind, oder ein Ester davon ist.

4. Pflaster nach Anspruch 1, wobei die Verbindung eine Verbindung der Formel (IV): 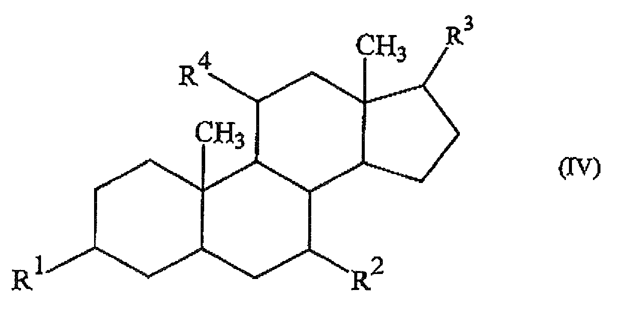 in der R¹, R², R³ und R⁴ wie in Anspruch 1 definiert sind, oder ein Ester davon ist.

5. Pflaster nach Anspruch 1, wobei die Verbindung 11β-Hydroxy-4-androsten-3,17-dion ist.

6. Pflaster nach Anspruch 1, wobei die Verbindung 7α-Hydroxy-dehydroepiandrosteron ist.

7. Pflaster nach Anspruch 1, wobei die Verbindung 7β-Hydroxy-epiandrosteron ist.

8. Pflaster nach einem der Ansprüche 1 bis 7, wobei die Menge an Einheiten, die von Methoxyethylacrylat abgeleitet sind, in dem Copolymer 45 bis 55 Gew.-% ist.

9. Pflaster nach einem der Ansprüche 1 bis 8, wobei das polare Monomer Acrylsäure, Methacrylsäure, Acrylamid, Methacrylamid, N-Vinyl-2-pyrrolidon, N,N-Dimethylacrylamid, 2-Hydroxyethylacrylat oder Vinylacetat ist.

10. Pflaster nach Anspruch 9, wobei das polare Monomer Acrylsäure, N-Vinyl-2-pyrrolidon oder 2-Hydroxyethylacrylat ist.

11. Pflaster nach Anspruch 10, wobei das polare Monomer N-Vinyl-2-pyrrolidon ist.

12. Pflaster nach einem der Ansprüche 1 bis 11, wobei der Klebstoff zusätzlich einen Absorptionspromotor enthält.

13. Pflaster nach Anspruch 12, wobei der Absorptionspromotor Isopropylmyristat ist.

14. Pflaster nach Anspruch 12 oder Anspruch 13, wobei der Absorptionspromotor in einer Menge von 3 bis 40 Gewichtsteile pro 100 Gewichtsteile des Copolymers vorliegt.

15. Pflaster nach einem der Ansprüche 1 bis 14, wobei die Klebstoffschicht eine Dicke von 30 bis 120 µm hat.

16. Pflaster nach einem der Ansprüche 1 bis 15 zur Verwendung bei der Behandlung oder Prävention von Osteoporose, Osteogenese, neuronalem Zelltod oder Ischämie-induzierter Schädigung an peripheren Organen durch transdermale Applikation.

17. Klebezusammensetzung, umfassend einen Klebstoff, der wenigstens eine Verbindung der Formel (I) oder einen Ester davon darin dispergiert hat, wobei der Klebstoff, der wenigstens eine Verbindung der Formel (I) oder einen Ester davon darin dispergiert hat, wie in einem der Ansprüche 1 bis 14 definiert ist, zur transdermalen Verabreichung der Verbindung oder des Esters.

18. Klebezusammensetzung nach Anspruch 17 zur Verwendung bei der Behandlung oder Prävention von Osteoporose, Osteogenese, neuronalem Zelltod oder Ischämie-induzierter Schädigung an peripheren Organen durch transdermale Applikation.

## Revendications

1. Timbre adhésif pour l'administration percutanée d'un composé de formule (I) : 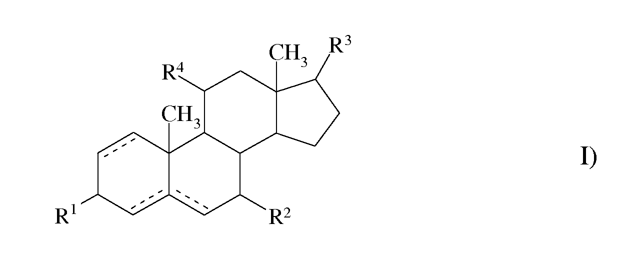 où R¹, R², R³ et R⁴ sont identiques ou différents les uns des autres et représentent chacun un groupe oxo, un groupe hydroxy, un groupe mercapto, un atome d'hydrogène, un atome d'halogène, un groupe alcoxy ou un groupe aryloxy ; et le trait en pointillés indique qu'il peut y avoir une liaison simple ou double entre l'une des paires d'atomes de carbone respectives, ou un de ses esters, ledit timbre comprenant un substrat et une couche d'adhésif déposée sur le substrat, ledit adhésif comprenant un copolymère qui contient de 40 à 60 % en poids d'acrylate de méthoxyéthyle, de 30 à 40 % en poids d'acrylate ou méthacrylate de lauryle et de 10 à 25 % en poids d'un monomère polaire, et au moins un composé de formule (I) ou un de ses esters étant dispersé dans ledit adhésif.

2. Timbre percutané selon la revendication 1, dans lequel ledit composé est un composé de formule (II) : 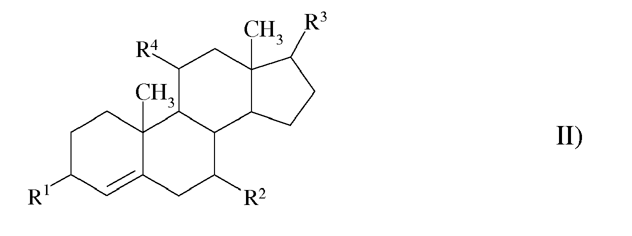 où R¹, R², R³ et R⁴ sont tels que définis dans la revendication 1, ou un de ses esters.

3. Timbre percutané selon la revendication 1, dans lequel ledit composé est un composé de formule (III) : 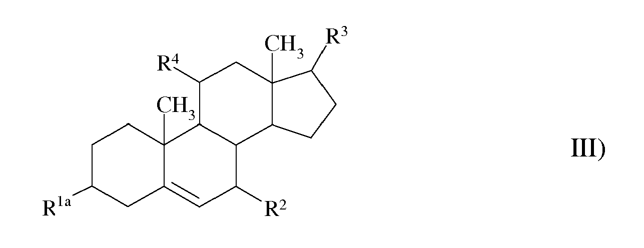 où R^{1a} représente un groupe oxo, un groupe hydroxy, un groupe mercapto ou un atome d'halogène ; et R², R³ et R⁴ sont tels que définis dans la revendication 1, ou un de ses esters.

4. Timbre percutané selon la revendication 1, dans lequel ledit composé est un composé de formule (IV) : 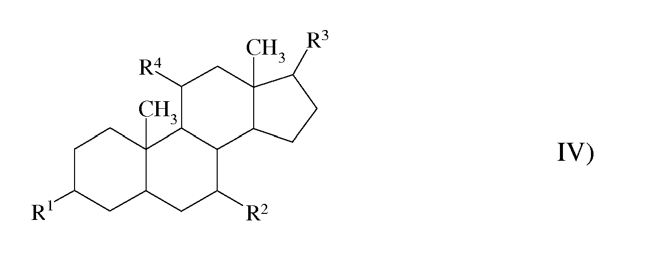 où R¹, R², R³ et R⁴ sont tels que définis dans la revendication 1, ou un de ses esters.

5. Timbre percutané selon la revendication 1, dans lequel ledit composé est la 11β-hydroxy-4-androstène-3,17-dione.

6. Timbre percutané selon la revendication 1, dans lequel ledit composé est la 7α-hydroxy-déshydroépiandrostérone.

7. Timbre percutané selon la revendication 1, dans lequel ledit composé est la 7β-hydroxy-épiandrostérone.

8. Timbre percutané selon l'une quelconque des revendications 1 à 7, dans lequel la quantité de motifs dérivés de l'acrylate de méthoxyéthyle dans le copolymère va de 45 à 55 % en poids.

9. Timbre percutané selon l'une quelconque des revendications 1 à 8, dans lequel le monomère polaire est l'acide acrylique, l'acide méthacrylique, l'acrylamide, le méthacrylamide, la N-vinyl-2-pyrrolidone, le N,N-diméthylacrylamide, l'acrylate de 2-hydroxyéthyle ou l'acétate de vinyle.

10. Timbre percutané selon la revendication 9, dans lequel le monomère polaire est l'acide acrylique, la N-vinyl-2-pyrrolidone ou l'acrylate de 2-hydroxyéthyle.

11. Timbre percutané selon la revendication 10, dans lequel le monomère polaire est la N-vinyl-2-pyrrolidone.

12. Timbre percutané selon l'une quelconque des revendications 1 à 11, dans lequel l'adhésif contient en plus un agent favorisant l'absorption.

13. Timbre percutané selon la revendication 12, dans lequel ledit agent favorisant l'absorption est le myristate d'isopropyle.

14. Timbre percutané selon la revendication 12 ou la revendication 13, dans lequel ledit agent favorisant l'absorption est présent en une quantité allant de 3 à 40 parties en poids pour 100 parties en poids du copolymère.

15. Timbre percutané selon l'une quelconque des revendications 1 à 14, dans lequel la couche d'adhésif a une épaisseur de 30 à 120 µm.

16. Timbre percutané selon l'une quelconque des revendications 1 à 15, destiné à être utilisé pour le traitement ou la prévention par voie percutanée de l'ostéoporose, de l'ostéogenèse, de la mort cellulaire neuronale ou de lésions des organes périphériques dues à une ischémie.

17. Composition adhésive, comprenant un adhésif dans lequel au moins un composé de formule (I) ou un de ses esters est dispersé, dans laquelle ledit adhésif dans lequel au moins un composé de formule (I) ou un de ses esters est dispersé est tel que défini dans l'une quelconque des revendications 1 à 14, pour l'administration percutanée dudit composé ou ester.

18. Composition adhésive selon la revendication 17, destinée au traitement ou à la prévention par voie percutanée de l'ostéoporose, de l'ostéogenèse, de la mort cellulaire neuronale ou de lésions des organes périphériques dues à une ischémie.
